# FASCICULE DE BREVET EUROPEEN

(11) **EP 0 857 073 B1**
(45) Date de publication et mention de la délivrance du brevet: **03.12.2003**
(21) Numéro de dépôt: 96934927.3
(22) Date de dépôt: 16.10.1996
(51) Int. Cl.: A61M 1/08, A61M 1/00

(54) **SERINGUE D'ASPIRATION ET DISPOSITIF D'ASPIRATION COMPRENANT UNE TELLE SERINGUE**
SPRITZE ZUM ANSAUGEN SOWIE SAUGVORRICHTUNG MIT EINER SOLCHEN SPRITZE
SUCTION SYRINGE AND SUCTION DEVICE COMPRISING SAME

(30) Priorité: 17.10.1995 FR 9512161
(43) Date de publication de la demande: 12.08.1998
(73) Titulaire: ASPIR, 95110 Sannois (FR)
(72) Inventeur: Emerit, Michel, 95110 Sannois (FR)
(74) Mandataire: Jacobson, Claude
(86) Numéro de dépôt international: FR9601623
(87) Numéro de publication internationale: WO97014452

(56) Documents cités:
- EP-A- 0 208 975
- WO-A-86/04819
- FR-A- 2 554 002
- FR-A- 2 574 299
- FR-A- 2 626 179
- GB-A- 2 008 200
- US-A- 4 116 240

## Description

La présente invention est relative à une seringue d'aspiration.

Des seringues d'aspiration, destinées en particulier à l'aspiration sur soi-même du venin d'une morsure ou d'une piqûre, ont été proposées dans le FR-A-2 408 738.

Ces seringues ont rencontré un important succès commercial, mais sont des dispositifs relativement sophistiques, qui comprennent un nombre important de pièces et sont relativement coûteux à fabriquer.

L'invention a pour but de fournir une seringue d'aspiration beaucoup plus économique, conçue pour être jetée après usage.

A cet effet, la seringue d'aspiration suivant l'invention est telle que définie dans la revendication 1.

Des modes de réalisation de l'invention sont définis dans les revendications 2 à 4.

L'invention a également pour objet un dispositif d'aspiration suivant la revendication 6, et dont plusieurs variantes sont définies dans les revendications 7 à 10.

Des exemples de réalisation de l'invention vont maintenant être décrits en regard des dessins annexés, sur lesquels :
- la Figure 1 est une vue en coupe longitudinale d'une seringue d'aspiration suivant l'invention, en position de stockage;
- la Figure 2 est une vue analogue illustrant la première phase d'utilisation de cette seringue;
- la Figure 3 est une vue analogue illustrant la deuxième phase d'utilisation de la seringue;
- la Figure 4 représente en coupe longitudinale un embout destiné à s'adapter sur la seringue d'aspiration des Figures 1 à 3;
- la Figure 5 est une vue du même embout, prise en coupe suivant la ligne V-V de la Figure 4;
- la Figure 6 est une vue analogue à la Figure 4 d'une variante d'embout; et
- les Figures 7 à 9 sont des vues analogues respectivement aux Figures 1 à 3 mais relatives à une variante de la seringue.

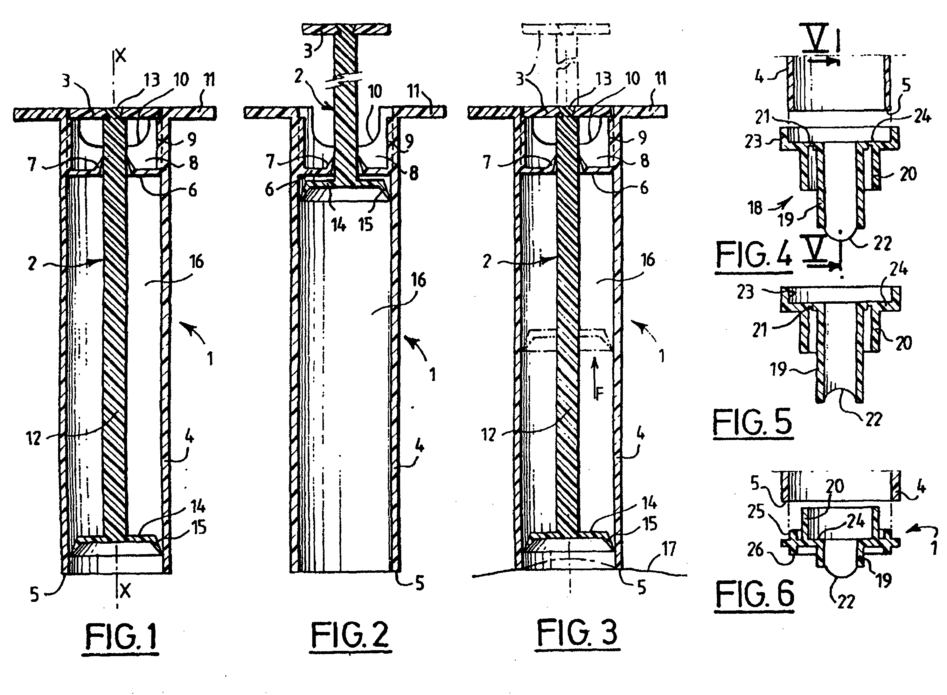

La seringue d'aspiration représentée à la Figure 1 est constituée de trois pièces moulées en matière plastique, à savoir un corps tubulaire 1, un ensemble tige-piston 2 et un bouton d'actionnement 3.

Le corps tubulaire 1 forme sur l'essentiel de sa longueur une enveloppe cylindrique 4 à section circulaire, d'épaisseur de paroi constante, intérieurement lisse sur toute sa longueur, ouverte à son extrémité distale de façon à définir une tranche d'extrémité plane et circulaire 5. Pour la commodité de la description, on supposera la seringue orientée comme représentée, c'est-à-dire avec son axe général X-X vertical et son extrémité distale en bas.

A son extrémité supérieure, l'enveloppe 3 porte un fond horizontal 6. Ce fond présente un orifice central délimité par une lèvre annulaire convergente 7 en saillie vers le haut.

Au-dessus du fond 6, l'enveloppe 4 se prolonge en un collet supérieur 8 de même section, muni intérieurement de deux nervures verticales 9 diamétralement opposées, lesquelles se terminent à une petite distance de l'extrémité supérieure du collet. Le collet 8 comporte par ailleurs, à 90° de ces nervures, deux échancrures 10, et, au droit des nervures, deux oreilles de préhension 11 en saillie radiale vers l'extérieur. Ces oreilles sont horizontales et affleurent l'extrémité supérieure du collet 8.

L'ensemble tige-piston 2 est constitué d'une tige pleine 12 qui traverse à joint étanche la lèvre 7 et qui porte à son extrémité supérieure un téton 13 d'encliquetage. L'extrémité inférieure de cette tige forme un piston plan 14 qui porte à sa périphérie une jupe 15 divergente dirigée vers le bas. La tige 12 coopère à joint étanche avec la lèvre 7, et la jupe 15 coopère à joint étanche avec la paroi intérieure lisse de l'enveloppe 4.

Le bouton 3 est plan et horizontal et est pourvu d'un orifice central qui s'encliquette sur le téton 13 de la tige 12.

Lors du montage de la seringue, la tige 12 est introduite par en bas dans le corps 1, traverse à frottement la lèvre 7 et est poussée vers le haut jusqu'à ce que la jupe 15 se trouve entièrement à l'intérieur de l'enveloppe 4. Le téton 13 se trouve alors à peu près au niveau des oreilles 11, et le bouton 3 est encliqueté sur ce téton. Sa périphérie vient alors reposer sur l'extrémité supérieure des nervures 9, et la face supérieure du bouton affleure celle des oreilles 11.

Dans cette position de stockage, une pression sensiblement égale à la pression atmosphérique règne dans la chambre annulaire 16 délimitée par l'enveloppe 4, le fond 6, la tige 12 et le piston 14.

Pour utiliser la seringue, on saisit le corps 1 d'une main, on passe deux doigts dans les échancrures 10, et on soulève le bouton 3. Ceci entraîne vers le haut l'ensemble 2-3, et l'air contenu dans la chambre 16 est chassé par décollement élastique de la lèvre 7 par rapport à la tige 12 et de la jupe 15 par rapport à l'enveloppe 4.

On arrive ainsi à la position stable de la Figure 2, où le piston 14 se trouve juste au-dessous du fond 6.

La suite de l'utilisation de la seringue s'effectue avec une seule main : on la saisit avec deux doigts sous les oreilles 11 et avec le pouce sur le bouton 3, et on fait descendre ce dernier.

Pendant ce mouvement, la lèvre 7 et la jupe 15 font étanchéité par effet de clapet, de sorte qu'une dépression croissante s'établit dans la chambre 16, jusqu'à un maximum correspondant au retour de l'ensemble 2-3 dans sa position initiale, définie par la butée du bouton 3 sur les nervures 9.

Toutefois, les positions des Figures 1 et 3 diffèrent par la pression qui règne dans la chambre 16.

On applique alors la tranche d'extrémité inférieure 5 sur la peau 17 autour de la piqûre ou de la morsure (Figure 3), et on relâche le bouton 3. Du fait de la dépression régnant dans la chambre 16, le piston 14 remonte de lui-même (flèche F), ce qui crée une dépression entre ce piston et la peau. Cette dernière est ainsi aspirée vers le haut, comme illustré en traits mixtes sur la Figure 3, ce qui provoque l'évacuation du venin.

Il est à noter que, si nécessaire, on peut décoller la tranche 5 de la peau, enfoncer de nouveau le bouton, et appliquer de nouveau la tranche 5 sur la peau, pour renforcer l'effet d'aspiration. Toutefois, il n'est normalement plus possible de rétablir la pression atmosphérique dans la chambre 16, de sorte que la seringue doit en principe être jetée après usage.

On peut compléter la seringue par un jeu d'embouts interchangeables emboîtables sur ou dans l'extrémité inférieure de l'enveloppe 4. En variante, comme représenté sur les Figures 4 et 5, on peut utiliser au moins un embout 18 équipé de deux conduits concentriques 19 et 20, le conduit le plus long, qui est dans cet exemple le conduit intérieur 19, pouvant être éliminé par rupture d'une zone fragile 21 prévue à sa base. Dans cet exemple, le conduit 19 possède une extrémité libre 22 en forme de selle de cheval, adapté pour épouser la forme d'un doigt, notamment d'un doigt d'enfant.

L'embout 18 s'emboît à joint étanche sur l'extrémité inférieure de l'enveloppe 4 au moyen d'un collet supérieur 23, légèrement divergent, qui borde la périphérie de son fond 24. Dans la variante de la Figure 6, au contraire, l'embout s'emboîte à joint étanche dans cette extrémité inférieure, au moyen d'un collet supérieur 25 décalé radialement par rapport à la périphérie du fond 24 et de forme légèrement convergente.

De plus, dans le cas de la Figure 6, il est prévu un second collet 26, symétrique du collet 25 par rapport au fond 24, et les conduits d'aspiration 19 et 20 font saillie l'un vers le bas et l'autre vers le haut sur ce fond.

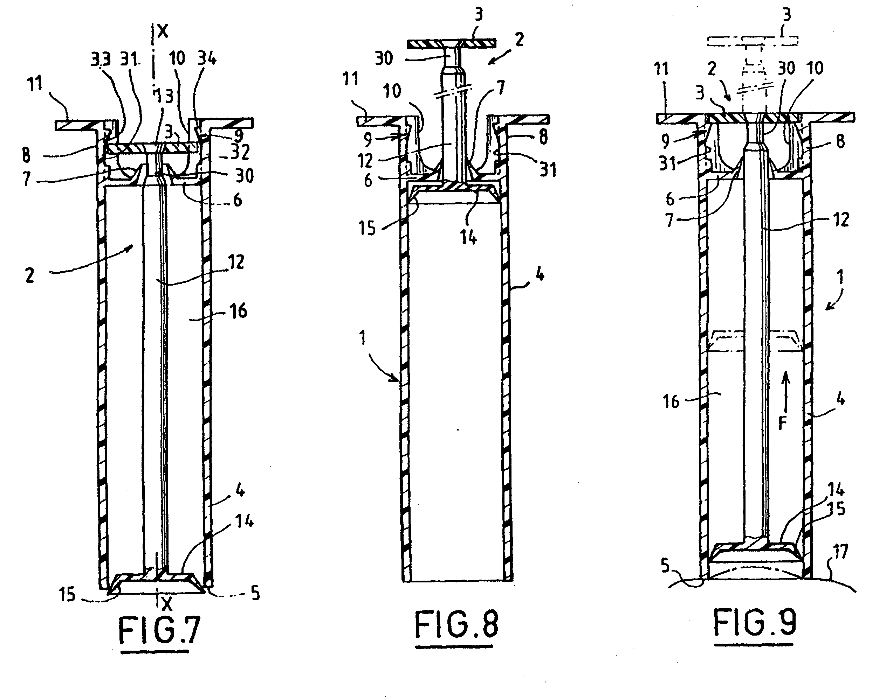

La variante de seringue des Figures 7 à 9 diffère de la précédente par les points suivants.

D'une part, la partie supérieure 30 de la tige 12, située immédiatement sous le bouton 3, a un diamètre réduit.

D'autre part, chaque nervure 9 comporte à un niveau intermédiaire un évidement 31 délimité vers le bas par une face horizontale 32 et vers le haut par une rampe 33 qui converge vers le haut. Cette dernière rejoint la face d'extrémité supérieure horizontale 34 de la nervure 9, de façon à former une dent.

Dans la position de stockage de la seringue (Figure 7), le bouton 3 est disposé dans l'évidement 31. Ceci amène la section rétrécie 30 de la tige 12 en regard de la lèvre 7 et l'extrémité libre de la jupe 15 au-delà de l'extrémité inférieure 5 du corps 1. Ces deux joints 7, 15 conservent par suite leur efficacité même après un stockage prolongé.

Pour utiliser la seringue, l'opérateur glisse deux doigts sous le bouton 3 à travers les encoches 10 et soulève ce bouton. L'élasticité du collet 8 et les rampes 33 permettent alors au bouton de passer au-dessus des nervures 9.

Puis, comme décrit plus haut en regard des Figures 2 et 3, l'ensemble bouton 3-tige 12 est tiré au maximum vers le haut (Figure 8). Le début de ce mouvement provoque la déformation des joints 7 et 15 par la tige 12 et par l'enveloppe 4 respectivement.

Puis le bouton 3 est enfoncé de nouveau (Figure 9) pour créer le vide dans le corps 1. Ce dernier mouvement est arrêté positivement par la butée du bouton 3 sur les faces supérieures 34 des nervures 9, de sorte que l'étanchéité est conservée en permanence au niveau des deux joints.

En variante, dans la position de stockage, la lèvre 15 pourrait se trouver, sans contact, en regard d'un évidement intérieur de l'enveloppe 4.

## Revendications

1. Seringue d' aspiration, comprenant trois pièces :
(a) un corps tubulaire (1) moulé en matière plastique, définissant sur l'essentiel de sa longueur une enveloppe (4) intérieurement lisse, cette enveloppe étant ouverte à une extrémité (5) et comportant à son autre extrémité un fond (6), ce fond présentant un orifice central délimité par une lèvre annulaire convergente (7) dirigée axialement vers l'extérieur de l'enveloppe, le corps (1) comportant deux oreilles de préhension (11) en saillie radiale vers l'extérieur;
(b) un ensemble tige-piston (2) moulé en matière plastique, comportant d'une part une tige (12), pleine ou dépourvue de conduit traversant, qui traverse l'orifice dudit fond (6) et coopère à joint étanche avec la lèvre annulaire (7), et d'autre part, à l'extrémité distale de cette tige, un piston (14) pourvu d'une jupe périphérique divergente (15) dirigée vers l'extrémité ouverte de l'enveloppe (4) et coopérant à joint étanche avec la paroi intérieure lisse de cette dernière; et
(c) un bouton d'actionnement (3) fixé à l'extrémité proximale de la tige (12), et
**caractérisée en ce que** la seringue est constituée desdites trois pièces (1, 2, 3), et **en ce que** le corps (1) est muni de moyens de butée (9) avec lesquels le bouton (3) est adapté pour venir en contact lorsque la jupe (15) du piston (14) se trouve au voisinage de l'extrémité ouverte (5) de l'enveloppe (4) et en contact avec celle-ci.

2. Seringue d'aspiration suivant la revendication 1, **caractérisée en ce que** le corps (1) comporte à son extrémité proximale, au-delà du fond (6), un prolongement (8) qui définit deux encoches (10) diamétralement opposées.

3. Seringue d'aspiration suivant la revendications 1 ou 2, **caractérisée en ce qu'**une partie d'extrémité proximale (30) de la tige (12) a une section transversale rétrécie, et **en ce que** le bouton (3) et le corps (1) comportent des moyens de positionnement relatif (31) qui placent cette partie d'extrémité en regard de la lèvre (7) du fond (6), sans contact avec celle-ci.

4. Seringue d'aspiration suivant l'une quelconque des revendications 1 à 3, **caractérisée en ce que** le bouton (3) et le corps (1) comportent des moyens de positionnement relatif (31) qui placent l'extrémité de la jupe (15) du piston (14) au-delà de l'extrémité ouverte de l'enveloppe (4) ou en regard d'un évidement interne de celle-ci et sans contact avec elle.

5. Seringue d'aspiration suivant la revendication 3 ou 4, **caractérisée en ce que** les moyens de positionnement relatif (31) comprennent un évidement du corps (1) plus proche du fond (6) que lesdits moyens de butée (9), et une dent (33-34) qui interdit le retour du bouton (3) dans cet évidement (31) après qu'il a franchi lesdits moyens de butée (9).

6. Dispositif d'aspiration, comprenant :
- une seringue (1 à 3) suivant l'une quelconque des revendications 1 à 5; et
- au moins un embout (18) en matière plastique moulée adapté pour s'emboîter à joint étanche à l'extrémité ouverte de l'enveloppe (4) de la seringue.

7. Dispositif d'aspiration suivant la revendication 6, **caractérisé en ce que** l'embout (18) comporte deux conduits d'aspiration (19, 20) de configurations différences, et des moyens (21; 25, 26) pour mettre en service l'un ou l'autre de ces conduits.

8. Dispositif d'aspiration suivant la revendication 7, **caractérisé en ce que** les deux conduits (19, 20) sont en saillie sur le côté distal de l'embout (18), l'un au moins des deux conduits pouvant être éliminé grâce à une zone fragile (21) qui le relie au reste de l'embout.

9. Dispositif d'aspiration suivant la revendication 6, **caractérisé en ce que** les deux conduits (19, 20) sont respectivement en saillie sur les deux côtés opposés de l'embout (18), lequel comporte des moyens (25, 26) d'emboîtement dans un sens ou dans l'autre à l'extrémité ouverte (5) de l'enveloppe (4).

10. Dispositif d'aspiration suivant l'une quelconque des revendications 6 à 8, comprenant au moins deux embouts interchangeables ayant des configurations d'aspiration différentes.

## Patentansprüche

1. Saugspritze, drei Teile umfassend:
(a) einen aus Kunststoff geformten rohrförmigen Körper (1), der über den wesentlichen Teil seiner Länge einen innen glatten Mantel (4) definiert, wobei dieser Mantel an einem Ende (5) offen ist und an seinem anderen Ende einen Boden (6) umfasst, dieser Boden eine Mittelöffnung aufweist, die durch eine ringförmige konvergierende axial nach außen bezüglich des Mantels gerichtete Lippe (7) begrenzt ist, wobei der Körper (1) zwei radial nach außen hervorspringende Greiflappen (11) umfasst;
(b) eine aus Kunststoff geformte Anordnung Stab-Kolben (2), die einerseits einen vollen oder keinen durchquerenden Kanal aufweisenden Stab (12), der Öffnung des Bodens (6) durchquert und mit dichtender Stoßfuge der ringförmigen Lippe (7) zusammenarbeitet, und andererseits am distalen Ende dieses Stabes einen Kolben (14) umfasst, der mit einer divergierenden, zum offenen Ende des Mantels (4) gerichteten Umfangsschürze (15) versehen ist und mit dichtender Stoßfuge mit der glatten Innenwand des Mantels zusammenarbeitet und
(c) einen Betätigungskopf (3), der am proximalen Ende des Stabes (12) befestigt ist und
**dadurch gekennzeichnet, dass** die Spritze aus den drei Teilen (1, 2, 3) besteht und dass der Körper (1) mit Anschlagsmittel (9) ausgerüstet ist, mit denen der Knopf (3) zusammenpasst, um in Kontakt zu kommen, wenn die Schürze (15) des Kolbens (14) sich in der Nähe des offenen Endes (5) des Mantels (4) und in Kontakt mit diesem befindet.

2. Saugspritze nach Anspruch 1, **dadurch gekennzeichnet, dass** der Körper (1) an seinem proximalen Ende, jenseits des Bodens (6) eine Verlängerung (8) aufweist, die zwei diametral entgegengesetzte Vertiefungen (10) definiert.

3. Saugspritze nach Anspruch 1 oder Anspruch 2, **dadurch gekennzeichnet, dass** ein Bereich des proximalen Endes (30) des Stabes (12) einen eingeschnürten Querschnitt aufweist und dass der Knopf (3) und der Körper (1) Mittel zur relativen Positionierung (31) umfassen, die diesen Endbereich gegenüberstehend zur Lippe (7) des Bodens (6) ohne Kontakt mit dieser platzieren.

4. Saugspritze nach einem der Ansprüche 1 bis 3, **dadurch gekennzeichnet, dass** der Knopf (3) und der Körper (1) Mittel zur relativen Positionierung (31) umfassen, die das Ende der Schürze (15) des Kolbens (14) jenseits des offenen Endes des Mantels (4) oder gegenüberstehend zu einer inneren Freimachung desselben und ohne Kontakt mit ihm plazieren.

5. Saugspritze nach Anspruch 3 oder 4, **dadurch gekennzeichnet, dass** die Mittel zur relativen Positionierung (31) eine Aussparung des Körpers (1), die näher zum Boden liegt, als die Anschlagmittel (9) und einen Zahn (33-34) umfassen, der die Rückkehr des Knopfes (3) in diese Aussparung (31) verhindert, nachdem er die Anschlagmittel (9) überschritten hat.

6. Saugvorrichtung umfassend:
- eine Spritze (1 bis 3) nach einem beliebigen der Ansprüche 1 bis 5 und
- mindestens einen Ansatz (18) aus geformten Kunststoffmaterial, der ausgebildet ist, um sich mit dichtender Stoßfuge in das offene Ende des Mantels (4) der Spritze einzufügen.

7. Saugvorrichtung nach Anspruch 6, **dadurch gekennzeichnet, dass** der Ansatz (18) zwei Saugkanäle (19, 20) mit unterschiedlicher Ausbildung und Mittel (21; 25, 26) umfasst, um den einen oder den anderen der Kanäle in Betrieb zu nehmen.

8. Saugvorrichtung nach Anspruch 7, **dadurch gekennzeichnet, dass** die zwei Kanäle (19, 20) auf der distalen Seite des Ansatzes (18) hervorspringen, wobei mindestens einer der zwei Kanäle weggenommen werden kann, dank einer zerbrechlichen Zone (21), die ihn mit dem Rest des Ansatzes verbindet.

9. Saugvorrichtung nach Anspruch 6, **dadurch gekennzeichnet, dass** die zwei Kanäle (19, 20) jeweils an den beiden gegenüberliegenden Seiten des Ansatzes (18) hervorragen, der Mittel (25, 26) zum Einstecken in eine oder die andere Richtung in das offene Ende (5) des Mantels (4) umfasst.

10. Saugvorrichtung nach einem der Ansprüche 6 bis 8, mindestens zwei auswechselbare Ansätze mit unterschiedlichen Saugkonfigurationen umfassend.

## Claims

1. Suction syringe comprising three parts:
(a) a tubular body (1) moulded from plastic material, defining over the major proportion of its length an envelope (4) that is smooth on the inside, this envelope being open at one end (5) and having a base (6) at its other end, this base having a central hole delimited by a convergent annular lip (7) directed axially towards the outside of the envelope, the body (1) having two gripping lobes (11) projecting radially outwards;
(b) a rod-piston assembly (2) moulded from plastic material, comprising, on the one hand, a rod (12), which may be solid or without a channel through it, which passes through the hole in the said base (6) and interacts with the annular lip (7) to give a tight joint, and, on the other hand, at the distal end of this rod, a piston (14) provided with a divergent peripheral skirt (15) directed towards the open end of the envelope (4) and interacting with the smooth interior wall of the latter to give a tight joint; and
(c) an actuating button (3) fixed to the proximal end of the rod (12), and
**characterised in that** the syringe is made up of the said three parts (1, 2, 3) and **in that** the body (1) is equipped with stop means (9) with which the button (3) is suitable for coming into contact when the skirt (15) of the piston (14) is in the vicinity of the open end (5) of the envelope (4) and in contact with the latter.

2. Suction syringe according to Claim 1, **characterised in that** at its proximal end, beyond the base (6), the body (1) has an extension (8) which defines two diametrically opposed recesses (10).

3. Suction syringe according to Claims (sic) 1 or 2, **characterised in that** a proximal end part (30) of the rod (12) has a narrowed cross-section and **in that** the button (3) and the body (1) have relative positioning means (31) which position this end part opposite the lip (7) of the base (6), without being in contact with the said lip.

4. Suction syringe according to any one of Claims 1 to 3, **characterised in that** the button (3) and the body (1) have relative positioning means (31) which position the end of the skirt (15) of the piston (14) beyond the open end of the envelope (4) or opposite an internal recess of the latter and without being in contact with it.

5. Suction syringe according to Claim 3 or 4, **characterised in that** the relative positioning means (31) comprise a recess in the body (1) closer to the base (6) than the said stop means (9) and a tooth (33-34) which prevents the button (3) from returning into this recess (31) after it has passed over the said stop means (9).

6. Suction device, comprising:
- a syringe (1 to 3) according to any one of Claims 1 to 5; and
- at least one end piece (18) made of moulded plastic material and suitable for fitting on the open end of the envelope (4) of the syringe to give a tight joint.

7. Suction device according to Claim 6, **characterised in that** the end piece (18) has two suction channels (19, 20) of different configurations and means (21; 25, 26) for bringing one or other of these channels into service.

8. Suction device according to Claim 7, **characterised in that** the two channels (19, 20) project on the distal side of the end piece (18), it being possible for at least one of the two channels to be removed by virtue of a fragile zone (21) which joins it to the remainder of the end piece.

9. Suction device according to Claim 6, **characterised in that** the two channels (19, 20) project, respectively, on the two opposite sides of the end piece (18), which contains means (25, 26) for fitting in one direction or in the other on the open end (5) of the envelope (4) .

10. Suction device according to any one of Claims 6 to 8, comprising at least two interchangeable end pieces having different suction configurations.
